# EUROPEAN PATENT APPLICATION

(11) **EP 1 106 194 A1**
(43) Date of publication of application: **13.06.2001**
(21) Application number: 99500232.6
(22) Date of filing: 03.12.1999
(51) Int. Cl.: A61M 5/50

(54) **Single-use disposable syringe**

(71) Applicant: Euro-China Farma, S.L., 46240 Carlet (Valencia) (ES)
(72) Inventor: Rico Bautista, Leonardo, 46240 Carlet (Valencia) (ES)
(74) Representative: Isern-Cuyas, Maria Luisa

(57) **Abstract**

The purpose of the invention is a single-use disposable syringe of the type used to inject liquid medicines, the structure of which consists of a hollow cylindrical body (1) and a plunger (4) that slides along its inside, operated by a rod (5).

The body (1) has elastic sheets (7) on its inside placed near the bottom and slanting towards it, which retain the plunger (4) when this goes past said sheets.

The union of the rod (5) with the plunger (4) is made by a protuberance (8) at the end of the rod, which is inserted in an unmovable way into the plunger so that, if an effort is made to extract the retained plunger, the union becomes broken.

Displacement of the plunger (4) can also be set after the first use by inserting the edge of the upper base of the rod (5) in a peripheral neck (12) placed on the inside surface of the body (1) near its mouth.

## Description

### PURPOSE

The purpose to which the invention protected by this Patent refers consists of a single-use disposable syringe of the type used for injecting liquid medicines in the tissues or organs.

### HISTORY

The known structure of these syringes includes:

A hollow cylindrical body which at one end has a tube of a smaller diameter in which a hollow needle with a sharp beveled point is inserted.

An elastic plunger which slides tightly along the cylindrical body to alternatively suck in and push the injectable liquid.

A rod which, attached by one end to the center of one of the faces of the plunger, sends it the movement by means of mechanical traction or compression stresses that are manually received at the opposite end provided with a circular flat base of larger diameter.

The syringes initially used had the same structure which allowed them to be disinfected in boiling water so that they could be used innumerable times.

Subsequent manufacture of moldable plastic syringes made it impossible for them to be sterilized as mentioned above and conditioned the prescription of one use only for plastic syringes.

However, the use of syringes by marginal groups has favored the contagion of illnesses transmitted by blood, as a result of the promiscuity of many persons using the same syringe even though, in spite of repeated warnings to the contrary in publicity campaigns, nothing prevents its multiple use.

### DESCRIPTION OF THE INVENTION

The object of the invention constituting the purpose of this Patent consists in overcoming the inconvenience itself of the known syringes described above, which were conceived and designed for this priority objective.

In effect, the foundation of the invention lies in providing known syringes with auxiliary means that make it technically impossible for them to be reused.

The invention includes two distinct possibilities, independent of the will of the user, that objectively prevent multiple use of the syringe. Said possibilities may be used alternatively or simultaneously, in the latter case as support or complementary safety.

The first option consists in providing the hollow body of the syringe with means to hold back the plunger, intended to prevent its upward movement once it has been pressed down, consisting of elastic sheets adhered to the inside surface of the body and arranged slightly slanting towards the bottom and at a distance slightly higher than the height of the plunger. These sheets may be one or more independent or one perimetral sheet only in the form of a circular crown.

The device is complemented by the characteristic insertion of the rod in the plunger, which is made up of a protuberance placed at the lower end of said plunger, with a semi-spherical or flat shape, joined to the rod by a plane parallel to the plane of the end by means of a thin and short neck.

The protuberance is inserted under pressure into the upper face of the plunger, which has a cavity of the same shape for this purpose, and is held in place thanks to the enclosure around the neck joining the rod of the thin elastic material sheets of the plunger that partially close the cavity.

Operation of the set is as follows: once the injection has been completed, the plunger will have reached the bottom of the syringe body, and is held there by the interior slanting sheets that act as a harpoon. If, in these conditions, an intent is made to return the plunger to its original position with the idea of reusing it by pulling it, the final protuberance of the cavity comes out from where it was housed and the syringe becomes unusable because it is not possible to re-insert said protuberance in the plunger.

According to another way of guaranteeing the impossibility of reusing the syringe, the length of the rod is adapted to that of the hollow body so that, when the plunger reaches the bottom of said body, the plane face of the upper circular base of the rod becomes level with its mouth, in order that its conveniently tapered edge is embedded in a perimetral neck placed on the inside surface of the hollow body, next to its mouth.

To facilitate this insertion, the lower face of the upper circular base of the rod has a convex shape towards the edge, so that it can be deformed in a more elastic way and overcome the projecting edges of the mouth until it is inserted from where it cannot be extracted.

This way of setting can be used together with the preceding one, as a safety reinforcement, or independently.

### BRIEF DESCRIPTION OF THE DRAWINGS

In order to complement the description of the invention and to make the interpretation of its formal, structural and functional characteristics easier, attached hereto are drawings in which different aspects of a preferred performance of the "single-use disposal syringe", the purpose of this Patent, are schematically shown.

In these drawings:

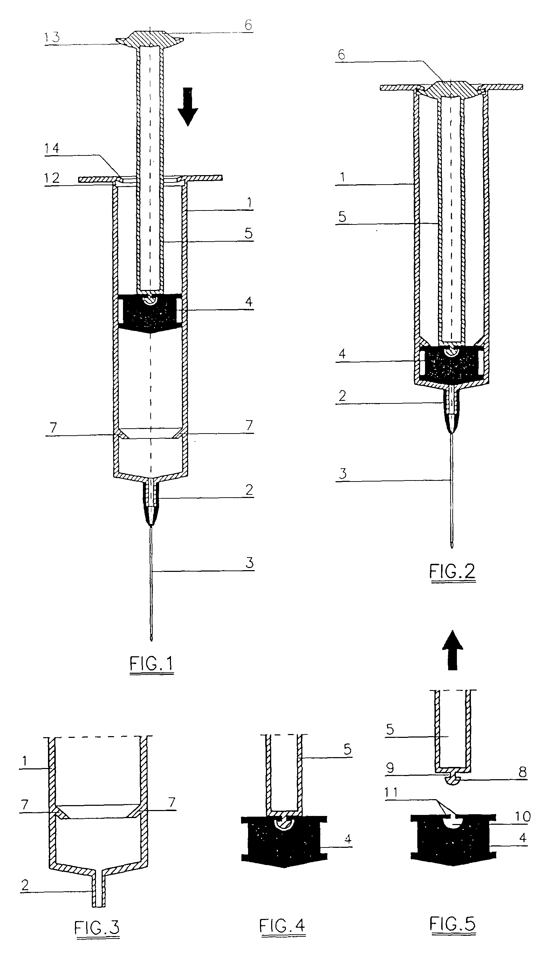

Figure 1 shows a longitudinal section of the syringe when giving an injection, whilst figure 2 corresponds to the same section when this movement is completed, with the plunger set at the bottom of the hollow body.

Figure 3 is a section detail of the arrangement of the plunger retaining elastic sheets.

Figure 4 shows the removable insertion form of the rod in the plunger, whilst Figure 5 shows how this link is unjoined when it is tried to extract the plunger to reuse the syringe. This operation is impossible to perform as the plunger is retained in an unmovable way at the bottom of the hollow body.

### DESCRIPTION OF A PREFERRED PERFORMANCE

In order to clearly show the nature and scope of the advantageous application of the "single-use disposable syringe", the purpose of this invention, its structure and operation are described below, making reference to the drawings that, on representing a preferred performance of said object for information purposes, must be considered in their widest sense and not as limiting the application and content of the invention.

The syringe is of the type used to inject liquid medicines in tissue or organs and whose known structure consists of:

A hollow cylindrical body (1) which at one end has a tube (2) of a smaller diameter in which a hollow needle (3) with a sharp beveled point is inserted.

An elastic plunger (4) which slides tightly along the cylindrical body (1) to alternatively suck in and push the injectable liquid.

A rod (5) which, attached by one end to the center of one of the faces of the plunger (4), sends it the movement by means of mechanical traction or compression stresses that are manually received at the opposite end provided with a circular flat base (6) of larger diameter.

In order to comply with its basic purpose of objectively preventing multiple use, independent of the intentions of the user, the hollow body (1) of the syringe is provided with means to retain the plunger (4) which are meant to prevent its retraction movement once it has been pressed down, which consist of elastic sheets (7) adhered to the inside surface of the body (1) and arranged slightly slanting towards the bottom and at a distance slightly higher than the height of the plunger (4). These sheets may be one or more independent or one perimetral sheet only in the form of a circular crown.

The union of the rod (5) with the plunger (4) is made by means of a protuberance (8) placed at the lower end of the rod, with a flat or semi-spherical shape, joined to the rod by a thin and short neck (9) that connects a maximum plane of said protuberance (8) with the end plane of the rod (5). Both planes are parallel.

The protuberance (8) is inserted under pressure into the upper face of the plunger (4), in a cavity (10) of the same shape, and the union is held in place by means of the enclosure around the neck (9) of the thin sheets (11) of the elastic material of the plunger (4) that partially close the cavity.

As the plunger (4) is held at the bottom of the body (1) by the slanting sheets (7), which act as a harpoon, if an effort is made to retract the rod (5) to its original position

its union with the plunger (4) is broken as the protuberance (8) leaves its housing in the cavity (10) and is not able to be reinserted.

According to another way of preventing the syringe from being reused, the length of the rod (5) is adapted to that of the hollow body (1) so that, when the plunger (4) reaches the bottom of said body (1), the plane face (6) of the upper circular base of the rod (5) becomes level with its mouth, in order that its outline is embedded in a perimetral neck (12) placed on the inside surface of the hollow body (1), next to its mouth.

To facilitate this insertion, the lower face (13) of the upper circular base of the rod (5) has a convex shape towards the edge, so that when said edge becomes thinner it can be deformed in a more elastic way and overcome the projecting edges (14) of the mouth until it is inserted in the neck (12) from where it cannot be extracted.

This way of setting can be used together with the preceding one, as a safety reinforcement, or independently.

## Claims

1. Single-use disposable syringe, of the type used to inject liquid medicines in tissue or organs, the structure of which is composed of:
A hollow cylindrical body (1) which at one end has a tube (2) of a smaller diameter in which a hollow needle (3) with a sharp beveled point is inserted.
An elastic plunger (4) which slides tightly along the cylindrical body (1) to alternatively suck in and push the injectable liquid.
A rod (5) which, attached by one end to the center of one of the faces of the plunger (4), sends it the movement by means of mechanical traction or compression stresses that are manually received at the opposite end provided with a circular flat base (6) of larger diameter, characterized basically by the fact that the hollow body (1) of the syringe is provided with means to retain the plunger (4), intended to prevent its retraction movement once it has been pressed down, which consist of elastic sheets (7) adhered to the inside surface of the body (1) and arranged slightly slanting towards the bottom and at a distance slightly higher than the height of the plunger (4). These sheets may be one or more independent or one perimetral sheet only in the form of a circular crown.

2. Single-use disposable syringe, according to claim 1, characterized basically by the fact that the union of the rod (5) with the plunger (4) is made by means of a protuberance (8) placed at the lower end of the rod, with a flat or semi-spherical shape, joined to the rod by a thin and short neck (9) that connects a maximum plane of said protuberance (8) to the end plane of the rod (5). Both planes are parallel. And also due to the fact that the protuberance (8) is inserted under pressure into the upper face of the plunger (4), in a cavity (10) of the same shape, and the union is held in place by means of the enclosure around the neck (9) of the thin sheets (11) of the elastic material of the plunger (4) that partially close the cavity. Therefore, as the plunger (4) is held at the bottom of the body (1) by the slanting sheets (7), which act as a harpoon, if an effort is made to retract the rod (5) to its original position its union with the plunger (4) is broken as the protuberance (8) leaves its housing in the cavity (10) and is not able to be reinserted.

3. Single-use disposable syringe, according to the above claims, characterized basically by the fact that the length of the rod (5) is adapted to the hollow body (1) so that, when the plunger (4) reaches the bottom of said body (1), the plane face (6) of the upper circular base of the rod (5) becomes level with its mouth, in order that its outline is embedded in a perimetral neck (12) placed on the inside surface of the hollow body (1), next to its mouth. To facilitate this insertion, the lower face (13) of the upper circular base of the rod (5) has a convex shape towards the edge, so that when said edge becomes thinner it can be deformed in a more elastic way and overcome the projecting edges (14) of the mouth until it is inserted in the neck (12) from where it cannot be extracted. This way of setting can be used together with the preceding one, as a safety reinforcement, or independently.
